# EUROPÄISCHE PATENTSCHRIFT

(11) **EP 1 072 604 B1**
(45) Veröffentlichungstag und Bekanntmachung des Hinweises auf die Patenterteilung: **07.03.2001**
(21) Anmeldenummer: 99250314.4
(22) Anmeldetag: 09.09.1999
(51) Int. Cl.: C07F 9/09, A61K 49/00, A61K 31/66, A61P 39/04

(54) **Calcium-Komplex von (4R)-4-bis(carboxy-kappa.O)methyl amino-.kappa.N-6,9-bis (carboxy-.kappa.O)methyl-1-(4,4-diphenylcyclohexyl)oxy-1-hydroxy-2-oxa-6,9-diaza-1-phosphaundecan-11-yl-säure-.kappa.N6,.kappa.N9,.kappa.O11 1-oxidato(6-)-,hexahydrogen, dessen Salze, diese Komplexe enthaltende pharmazeutische Mittel, deren Verwendung in der Therapie und als Zusatzstoff in der Diagnostik sowie Verfahren zur Herstellung der Komplexe und Mittel**
Calcium complex of (4R)-4-bis(carboxy-kappa.O)methyl amino-.kappa.N-6,9-bis (carboxy-.kappa.O)methyl-1-(4,4-diphenylcyclohexyl)oxy-1-hydroxy-2-oxa-6,9-diaza-1-phosphaundecan- 11-yl acid-.kappa.N6,.kappa.N9,.kappa.O11 1-oxidato(6-)-,hexahydrogen, corresponding salts, pharmaceutical composition containing such complex, its therapeutic use and its use as additive as diagnostic agent as well as a process for preparing the complex
Complexe de Calcium du (4R)-4-bis(carboxy-kappa.O)methyl amino-.kappa.N-6,9-bis (carboxy-.kappa.O)methyl-1-(4,4-diphenylcyclohexyl)oxy-1-hydroxy-2-oxa-6,9-diaza-1-phosphaundecan- 11-oique-.kappa.N6,.kappa.N9,.kappa.O11 1-oxidato(6-)-,hexahydrogen, leurs sels, les médicaments contenant ces complexes, leur utilisation thérapeutique et comme additifs dans des agents de diagnostic ainsi qu'un procédé de préparation du complexe

(43) Veröffentlichungstag der Anmeldung: 31.01.2001
(73) Patentinhaber: SCHERING AKTIENGESELLSCHAFT, 13353 Berlin (DE)
(72) Erfinder: Platzek, Johannes, Dr., 12621 Berlin (DE); Niedballa, Ulrich, Dr., 14195 Berlin (DE); Michl, Günter, Dr., 15568 Rüdersdorf (DE)

(56) Entgegenhaltungen:
- EP-A- 0 071 564
- WO-A-96/23526

## Beschreibung

Die Erfindung betrifft den in den Patentansprüchen gekennzeichneten Gegenstand, d.h. den Calcium-Komplex von[[(4R)-4-[bis[(carboxy-.kappa.O)methyl]amino-.kappa.N]-6,9-bis((carboxy-.kappa.O)methyl]-1-[(4,4-diphenylcydohexyl)oxy]-1-hydroxy-2-oxa-6,9-diaza-1-phosphaundecan-11-yl-säure .kappa.N6,.kappa.N9,.kappa.O11]1oxidato (6-)]-, hexahydrogen, dessen Salze, diese Komplexe enthaltende pharmazeutische Mittel, für die-Herstellung von Mitteln zur Verringerung von Wirkungen, die durch Schwermetalle verursacht werden sowie Verfahren zu deren Herstellung.
In der Medizin finden Komplexverbindungen ihren Einsatz vornehmlich für die Behandlung von Schwermetallvergiftungen, pathologischem Eisenüberschuß sowie zur Herstellung pharmazeutischer Mittel für die bildgebende Diagnostik.

In der EP 71564 wird u.a. das Megluminsalz des Gadolinium(III)-Komlexes der Diethytentriaminpentaessigsäure (DTPA) als Kontrastmittel für die NMR-Tomographie beschrieben. Ein Präparat, das diesen Komplex enthält, wurde unter dem Namen Magnevist® weltweit als erstes NMR-Kontrastmittel zugelassen. Dieses Kontrastmittel verteilt sich nach intravenöser Applikation extrazellulär und wird durch glomeruläre Sekretion renal ausgeschieden. Eine Passage intakter Zellmembranen wird praktisch nicht beobachtet .Magnevist® ist besonders gut für die Darstellung pathologischer Bereiche ( z.B. Entzündungen, Tumore) geeignet.

DTPA-bzw.Ca-DTPA-haltige Verbindungen finden weiterhin bei Metallvergiftungen ihre klinische Anwendung.

Es besteht aber weiterhin ein Bedarf an Mitteln zur Verringerung von Wirkungen, die durch Schwermetalle verursacht werden.

Der Erfindung liegt somit die Aufgabe zugrunde, derartige Verbindungen und Mittel zur Verfügung zu stellen, sowie ein Verfahren zu ihrer Herstellung zu schaffen. Die Lösung dieser Aufgabe erfolgt durch die vorliegende Erfindung.

In der Offenlegungsschrift WO 96/23526 wird die Verwendung des Gadolinato(3-),[[(4R)-4-[bis[(carboxy-.kappa.O)methyl]amino-.kappa.N]-6,9-bis[(carboxy-.kappa.O)methyl]-1-[(4,4-diphenylcydohexyl)oxy]-1-hydroxy-2-oxa-6,9-diaza-1-phosphaundecan-11-yl-säure-.kappa.N6,.kappa.N9.kappa.O11]1-oxidato(6-)]- trihydrogen, auch als Gd-MS-325 bezeichnet, als blood pool agent beschrieben. Gd-MS-325 zeichnet sich dadurch aus, daß es an Humanserumalbumin (HSA) bindet und dadurch im Intravasalraum verweilt. Die Herstellung einer Gd-MS-325-Formulierung ist ebenfalls in der WO 96/23526 beschrieben. So ist in Beispiel 10 die Herstellung einer 200 mM Lösung aus Gd-MS-325 (Meglumin-Salz) mit einem 5%igen Komplexbildnerüberschuß MS-325 beschrieben. Der Zusatz von Calciumsalzen zur Formulierung wird zwar im Anspruch 101 beansprucht, wird aber im Text und in den Beispielen nicht explizit offenbart.

In WO-96/23526 wird die Herstellung einer Gd-MS-325 Formulierung mit 5% Überschuß an Komplexbildner MS-325 beschrieben (Beispiel 10). Der Versuch, in dieser Formulierung den Ca-MS-325 Komplex herzustellen, war erfolglos (s. Versuche I-III).

So wurde versucht, den 5% igen Komplexbildnerüberschuß mit Ca-Hydroxid oder Ca-carbonat in situ zu komplexieren. Dabei traten, besonders bei größeren Ansätzen (1-10 I), Trübungen auf. Eine AAS-Analyse dieser Trübung ergab, daß es sich hierbei um Gd-haltige Komponenten handelt. (Wahrscheinlich bildet MS-325 während des Komplexierungsprozesses ein kinetisch bevorzugtes Zwischenprodukt, das auf Grund seiner Schwerlöslichkeit zur Trübung führt. Vermutlich sind es Ca-Komplexe des Phosphatesters. Die Tatsache, daß es sich um ein kinetisch bedingtes Zwischenprodukt handelt, wird dadurch bekräftigt, daß, wenn man eine derartige trübe Lösung 48 Stunden unter Rückfluß erhitzt, der Niederschlag sich auflöst und eine klare Lösung entsteht.) Außerdem wird mittels HPLC schon teilweise Zersetzung beobachtet. Auf Grund der abzufiltrierenden Trübung, deren Intensität teilweise noch davon abhängt wie schnell das Calcium zur Lösung zugesetzt wird, erhält man keine reproduzierbaren Gehalte an Ca-MS-325 und Gd-MS-325 in der Formulierung. Insgesamt ist somit dieses Vorgehen für die galenische Produktion derartiger pharmazeutischer Formulierungen nicht zulässig.

Es bestand daher auch die Aufgabe, eine den galenischen Anforderungen entsprechende Formulierung von Gd-MS-325 zur Verfügung zu stellen. Diese Aufgabe wird durch die vorliegende Erfindung gelöst.
Es wurde gefunden, daß die oben genannte Aufgabe durch die separate Herstellung des Ca-Komplexes des MS-325-Liganden und anschließender Zugabe zu der Gd-MS-325-Lösung gelöst wird.

Formulierungen, die auf diese Weise hergestellt werden, zeigen auch bei großen Ansätzen konstante und reproduzierbare analytische Daten. Außerdem weisen sie eine bessere Verträglichkeit, vollständigere Metallausscheidung und bessere Herzkreislaufeigenschaften als die ursprüngliche Gd-MS-325-Formulierung auf.

Die Erfindung betrifft daher den Calcium-Komplex von[[(4R)-4-[bis[(carboxy .kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-ylsäure-.kappa.N6,.kappa.N9,.kappa.O11]1-oxidato(6-)]-, hexahydrogen seine Herstellung und die Salze dieser Komplexe mit physiologisch verträglichen anorganischen und/oder organischen Kationen wie z.B. Natrium, Calcium, Kalium, Meglumin, Ethanolamin, Diethanolamin, Morpholin, Glucamin, Dimethylglucamin, Lysin, Arginin und loder Omithin, sowie die damit hergestellten galenischen Formulierungen mit Gd-MS-325.

Die Erfindung betrifft weiterhin die Verwendung von Ca-MS-325 und dessen Salzen zur Herstellung pharmazeutischer Mittel, insbesondere als Antidot gegen Schwermetallvergiftungen.

Herstellung der erfindungsgemäßen Verbindungen:

Der Komplexbildner wird durch Umsetzung mit Calciumhydroxidlösung, Calciumoxid, Calciumcarbonat oder Calciumbicarbonat in den Calciumkomplex (Ca-MS-325 ) überführt. Anschließend werden, falls gewünscht, vorhandene acide Wasserstoffatome von Säuregruppen durch Kationen anorganischer und / oder organischer Basen oder Aminosäuren substituiert.

Die Neutralisation erfolgt dabei mit Hilfe anorganischer Basen (z.B. Hydroxiden, Carbonaten, oder Bicarbonaten) von z.B. Natrium, Kalium, Lithium oder Calcium und / oder organischer Basen wie unter anderem primärer, sekundärer und tertiärer Amine, wie z.B. Ethanolamin, Glucamin, N-Methyl- und N,N-Dimethylglucamin sowie basischer Aminosäuren, wie z.B. Lysin, Arginin und Omithin.

Da der Ca-Komplex vier freie acide Gruppen enthält, kann es zweckmäßig sein, neutrale Mischsalze herzustellen, die sowohl anorganische als auch organische Kationen als Gegenionen enthalten.

Dies kann beispielsweise geschehen, indem man Ca-MS-325 in wäßriger Lösung mit dem Oxid oder Salz des gewünschten Metalls und gegebenenfalls der zur Neutralisation benötigten Menge einer anorganischen oder organischen Base umsetzt, das gebildete Komplexsalz isoliert und gewünschtenfalls aufreinigt. Die Reihenfolge der Basenzugabe ist beliebig.

Die Herstellung der erfindungsgemäßen Gd-MS-325-Formulierungen erfolgt, indem man die erfindungsgemäßen Calcium-Komplexverbindungen zusammen mit Gd-MS-325 - gegebenenfalls unter Zugabe der in der Galenik üblichen Zusätze - in wäßrigem Medium löst und anschließend die Lösung gegebenenfalls sterilisiert. Es kann auch vorteilhaft sein, diese so herzustellen, indem man die erfindungsgemäßen Calcium-Komplexverbindungen mit freiem Komplexbildner MS-325 und der stöchiometrischen Menge an Gadolinum-oxid bzw. -salz sowie der zur Neutralisation des Gadolinum-Komplexes benötigten Menge einer anorganischen oder organischen Base in wäßrigem Medium umsetzt. Geeignete Zusätze sind beispielsweise physiologisch unbedenkliche Puffer (wie zum Beispiel Tromethamin), Elektrolyte (wie zum Beispiel Natriumchlorid) sowie Antioxidantien (wie zum Beispiel Ascorbinsäure).

Die erfindungsgemäßen pharmazeutischen Mittel enthalten 1µmol/l - 1 Mol/l des Gd-Komplexsalzes, vorzugsweise 0,5mmol/l - 500mmol/l und 0,05 -15Mol%, bevorzugt 200 mmol/l und 0,5 - 5Mol%, Ca-MS-325 und werden in der Regel in Mengen von 0,005 - 2 mMol/kg Körpergewicht, vorzugsweise 50 µmol/kg - 500 µmol/kg dosiert.

Die folgenden Beispiele dienen zur näheren Erläuterung des Erfindungsgegenstandes.

Versuch der in situ-Herstellung von Gd-MS-325 mit 5Mol% Ca-MS-325
**I**
   Herstellung einer in situ- Formulierung von Gadolinium Komplex von MS-325 als Megluminsalz ( 200 mM ) mit 5 % Ca-MS-325 Überschusskomplexbildner
   181,25 g ( 0,5 mol ) Gadoliniumoxid, 815,35 g (1,05mol / 95 % Gehalt (nach Gewicht )MS-325 ( Ligand ) und 683,25 g ( 3,5 mol) N - Methylglucamin werden in 3500 ml deionisiertem Wasser gegeben und anschließend 3,70 g (50 mmol ) Calciumhydroxid zugegeben . Man rührt 6 Stunden bei 95°C. Es bildet sich eine trübe Lösung .Nach Abkühlen wird die Lösung auf ein Volumen von 5000 ml mit deionisiertem Wasser aufgefüllt und anschließend von der Trübung abfiltriert ( 2µ - Filter )
   Der Trübungsniederschlag wird in Vakuum ( 60°C ) getrocknet ( Ausbeute : 2,86 g ) und zur AAS - Analyse in Salpetersäure / Wasserstoffperoxid (Mikrowelle) gelöst. Es wurde ein Gd --Gehalt von 8,1% (bezogen auf Feststoff ) gefunden .
**II**
   Herstellung einer in situ- Formulierung von Gadoliniumkomplex von MS-325 als Megluminsalz ( 200 mM ) mit 5 % Ca-MS 325 Überschusskomplexbildner ). 181,25g ( 0,5 mol) Gadoliniumoxid, 815,35g (1,05mol / ca.95% Gehalt (nach Gewicht) MS-325 ( Ligand ) und 683,25 g ( 3,5 mol) N - Methylglucamin werden in 3500 ml deionisiertem Wasser gegeben und anschließend 5,00 g (50 mmol ) Calciumcarbonat zugegeben. Man rührt 6 Stunden bei 95°C. Es bildet sich eine trübe, undurchsichtige Lösung. Nach Abkühlen füllt man mit deionisiertem Wasser auf 5000 ml Gesamtvolumen auf. Und filtriert anschließend von der Trübung ab (2µ -Filter ). Der abfiltrierte
   Trübungsniederschlag wird getrocknet (60 ° C in Vakuum), Ausbeute : 3.14 g (Zur AAS - Analyse wird in Salpetersäure / Wasserstoffperoxid (Mikrowelle ) gelöst. Es wurde ein Gd - Gehalt von 9,6 % (bzg. auf Feststoff) gefunden.
**III**
   Herstellung einer in situ- Formulierung von Gadoliniumkomplex von MS-325 als Megluminsalz ( 200 mM ) mit 5 % Ca-MS 325 Überschusskomplexbildner ). 18,12 g ( 50 mmol ) Gadoliniumoxid , 81,54 g ( 105 mol / ca 95 % Gehalt ( nach Gewicht ) MS-325 Ligand und 68,3 g ( 350 mmol ) N-Methylglucamin werden in 350 ml deionisiertem Wasser gegeben und anschließend 0,37 g ( 5 mmol ) Calciumhydroxid zugegeben. Man erhitzt 48 Stunden unter Rückfluß (dabei klart die anfangs trübe farblose Lösung langsam auf / Farbe leicht gelb) man läßt abkühlen und füllt mit deionisiertem Wasser auf 500 ml Gesamtvolumen auf. Die leicht gelbe Lösung wird filtriert und durch HPLC analysiert. Der Gehalt an Gd-MS-325 wurde mit der 100 % - Methode bestimmt (externer Standard : HPLC - gereinigtes Gd-MS 325). Es ergab sich ein Gehalt von: 96,3 %. Der niedrige Gehalt und die Gelbfärbung deuten auf Zersetzung hin.
   Zum Vergleich : Gehalt nach HPLC / 95 °C (6 Stunden): 98,9 %-.

### Erfindungsgemäße Beispiele

### Beispiel 1

Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa. N6,.kappa.N9,.kappa.011]1-oxidato(6-)]-,
tetranatrium

10,0 g (12,88 mmol / 95 % Gehalt ( nach Gewicht ) ) MS-325 Ligand, 0,954g (12,88mmol) Calciumhydroxid und 1,546g ( 38,64 mmol ).
Natriumhydroxid werden in 2000 ml deionisiertem Wasser gelöst und 5 Stunden bei 95°C gerührt. Man läßt abkühlen und gibt weitere 0,515 g (12,88mmol) Natriumhydroxid zu, filtriert über ein 2µ Filter und isoliert das Titelprodukt aus dem Filtrat durch Gefriertrocknung als farbloses, amorphes Pulver.
Ausbeute : 12,40 g (quantitativ) Wassergehalt :10,3 %
Elementaranalyse (berechnet auf wasserfreie Substanz )
ber. C 45,89 / H 4,43 / N 4,87 / Ca 4,64 / Na 10,65 / P 3,59
gef. C 46,01 / H 4,52 / N 4,99 / Ca 4,53 / Na 10,77 / P 3,70

### Beispiel 2

Calcium(4-),[[(4R)-4.[bis[(carboxy-.kappa.O)methyl]amino-.kappa.N]-6,9-bis[(carboxy-.kappa.O)methyl]-1-[(4,4-diphenytcyclohexyl)oxy].1-hydroxy-2-oxa-6,9-diaza-1,phosphaundecan-11-yl-säure-.kappa.N6,.kappa.N9,.kappa.011]1-oxidato(6-)]-,
calcium, dinatrium
10,0 g (12,88 mmol / 95 % Gehalt ( nach Gewicht ) ) MS-325 Ligand,
2,578g(25,76 mmol) Calciumcarbonat und 0,515g ( 12,88 mmol )
Natriumhydroxid werden in 2000 ml deionisiertem Wasser gelöst und 5 Stunden bei 95°C gerührt. Man läßt abkühlen und gibt weitere 0,515g (12,88mmol) Natriumhydroxid zu, filtriert über ein 2p Filter und isoliert das Titelprodukt aus dem Filtrat durch Gefriertrocknung als farbloses, amorphes Pulver.
Ausbeute : 12,25 g (quantitativ) Wassergehalt :9,8 %
Elementaranalyse (berechnet auf wasserfreie Substanz )
ber. C 46,21 / H 4,47 */* N 4,90 */* Ca 9,34 / Na 5,36 / P 3,61
gef. C 46,32 / H 4,55 / N 5,00 / Ca 9,22 */* Na 5,45 / P 3,73

### Beispiel 3 :

Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa- 6,9-diaza-1-phosphaundecan-11-yl-säure-.kappa.N6,.kappa.N9,.kappa.011]1-oxidato(6-)]-,
dicalcium

10,0 g (12,88 mmol / 95 % Gehalt ( nach Gewicht ) ) MS-325 Ligand, 3,867g (38,64mmol) Calciumcarbonat werden in 200 ml deionisiertem Wasser gelöst und 5 Stunden bei 95°C gerührt. Man läßt abkühlen filtriert über ein 2µ Filter und isoliert das Titelprodukt aus dem Filtrat durch Gefriertrocknung als farbloses amorphes Pulver.
Ausbeute : 11,91 g (quantitativ) Wassergehalt :7,9 %
Elementaranalyse (berechnet auf wasserfreie Substanz )
ber. C 46,53 / H 4,50 / N 4,93 / Ca 14,11 / P 3,64
gef. C 46,41 / H 4,61 / N 5,02 / Ca 14,22 / P 3,75

### Beispiel 4

Calcium(4-),[[(4R)-4-[bis[(carboxy-.kappe.O)methyl]amino-.kappa.N]-6,9-bis[(carboxy-.kappe.O)methyl]-1,[(4,4-diphenylcydohexyl)oxy]-1-hydroxy-2-oxa-6,9-diaza-1-phosphaundecan-11-yl-säure-.kappa.N6,.kappa.N9,.kappa,011]1-oxidato(6-)]-,
tetrameglumin

10,0 g (12,88 mmol / 95 % Gehalt ( nach Gewicht ) ) MS-325 Ligand, 1,288g (12,88mmol) Calciumcarbonat und 8,80g (45,08 mmol Meglumin werden in 2000 ml deionisiertem Wasser gelöst und 5 Stunden bei 95°C gerührt. Man läßt abkühlen und gibt weitere 1,257g (6,44mmol) Meglumin zu , filtriert über ein 2p Filter und isoliert das Titelprodukt aus dem Filtrat durch Gefriertrocknung als farbloses amorphes Pulver.
Ausbeute : 22,27 g (quantitativ) Wassergehalt :10,0 %
Elementaranalyse (berechnet auf wasserfreie Substanz )
ber. C 47,07 / H 7,12 / N 6,30 / Ca 2,57 / P 1,99
gef. C 47,20 / H 7,21 / N 6,43 / Ca 2,69 / P 2,10

### Beispiel 5

Calcium(4-),[[(4R)-4-[bis[(carboxy-.kappa.O)methyl]amino-.kappa.N]-6,9-bis[(carboxy-.kappa.O)methyl]-1-[(4,4-diphenylcydohexyl)oxy]-1-hydroxy-2-oxa-6,9-diaza-1-phosphaundecan-11-yl-säure-.kappa.N6,.kappa.N9,.kappa.011]1-oxidato(6-)]-,
calcium , dimeglumin

10,0 g (12,88 mmol / 95 % Gehalt ( nach Gewicht ) ) MS-325 Ligand, 1,91g (25,76mmol) Calciumhydroxid und 3,77g (19,32) mmol Meglumin werden in 200 ml deionisiertem Wasser gelöst und 5 Stunden bei 95°C gerührt. Man läßt abkühlen und gibt weitere 1,257g (6,44mmol) Meglumin zu , filtriert über ein 2µ Filter und isoliert das Titelprodukt aus dem Filtrat durch Gefriertrocknung als farbloses amorphes Pulver.
Ausbeute : 17,06 g (quantitativ) Wassergehalt :9,1 %
Elementaranalyse (berechnet auf wasserfreie Substanz )
ber. C 46,88 / H 6,19 / N 5,82 / Ca 6,66 / P 2,57
gef. C 47,01 / H 6,29 / N 5,93 / Ca 6,58 / P 2,69

### Beispiel 6

Calcium(4-),[[(4R)-4-[bis[(carboxy-.kappa.O)methyl]amino-.kappa.N]-6,9-bis((carboxy-.kappa.O)methyl]-1-[(4,4-diphenylcydohexyl)oxy]-1-hydroxy-2-oxa-6,9-diaza-1-phosphaundecan-11-yl-säure-.kappa.N6,.kappa.N9,.kappa.011]1-oxidato(6-)]-,
calcium, dihydrogen

10,0 g (12,88mmol ) 95 % Gehalt (nach Gewicht) MS-325 Ligand 1,91 g (25,76 mmol )
Calciumhydroxid werden in 200 ml deionisiertem Wasser gelöst und 5 Stunden bei 95°C gerührt. Man läßt abkühlen, filtriert über ein 2µ Filter und isoliert das Titelprodukt aus dem Filtrat durch Gefriertrocknung als farbloses amorphes Pulver.
Ausbeute : 11,30 g (quantitativ ) Wassergehalt: 7,3 %
Elementaranalyse (berechnet auf wasserfreie Substanz)
ber.: C 51,0g / H 5,46 / N 5,42 / Ca 5,17/ P 3,99
gef: C 51,21 / H 5,55 / N 5,53 / Ca 5,08 / P 4,10

### Beispiel 7

Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
tetrahydrogen

10g (12,88 mmol ) MS-325 und 0.954 g ( 12,88mmol ) Calciumhydroxid werden in 100 ml Wasser gegeben und 1,55g (38,64 mmol ) Natriumhydroxid zugegeben. Man erwärmt 5 Stunden bei 80°C. Man kühlt auf 10°C ab und stellt mit 10 % iger aqu.Salzsäure auf pH 2,5. Anschließend gibt man 200 ml Isopropanol zu und kühlt auf 0°C ab. Man fällt 3 Stunden bei 0°C und filtriert anschließend vom ausgefallenen Niederschlag ab. Der abfiltrierte Niederschlag wird 2 mol mit 50 ml Ethanol und 2 mal mit 100 ml Diethylether gewaschen und im Vakuum getrocknet.
Ausbeute : 8,76g (87 % der Theorie ) eines farblosen kristallinen Pulvers .
Wassergehalt: 7,6 % Elementaranalyse (berechnet auf wasserfreie Substanz ):
ber./ C 51,09 / H 5,46 / N 5,42 / Ca 5,17 / P 3,99
gef./ C 50,87 / H 5,64 / N 5,28 / Ca 5,01 / P 3,72

### Beispiel 8

Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
dinatrium , dihydrogen

10,0 g (12,88 mmol / 95 % Gehalt ( nach Gewicht ) ) MS-325 Ligand, 1,288g (12,88mmol) Calciumcarbonat und 1,03g ( 25,76 mmol ) Natriumhydroxid werden in 200 ml deionisiertem Wasser gelöst und 5 Stunden bei 95°C gerührt . Man läßt abkühlen filtriert über ein 2µ Filter und isoliert das Titelprodukt aus dem Filtrat durch Gefriertrocknung als farbloses amorphes Pulver.
Ausbeute : 11,70 g (quantitativ) Wassergehalt :9,8 %
Elementaranalyse (berechnet auf wasserfreie Substanz )
ber. C 48,35 / H 4,92 / N 5,13 / Ca 4,89 / Na 5,61 / P 3,78
gef. C 48,49 / H 5,01 / N 5,24 / Ca 5,00 / Na 5,50 / P 3,90

### Beispiel 9

Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
trinatrium, monohydrogen

10,0 g (12,88 mmol / 95 % Gehalt ( nach Gewicht)) MS-325 Ligand, 0,954g (12,88mmol) Calciumhydroxid und 1,546g ( 38,64 mmol ) Natriumhydroxid werden in 200 ml deionisiertem Wasser gelöst und 5 Stunden bei 95°C gerührt . Man läßt abkühlen filtriert über ein 2µ Filter und isoliert das Titelprodukt aus dem Filtrat durch Gefriertrocknung als farbloses amorphes Pulver.
Ausbeute : 12,14 g (quantitativ) Wassergehalt :10,7 %
Elementaranalyse (berechnet auf wasserfreie Substanz )
ber. C 47,09 / H 4,67 / N 4,99 / Ca 4,76 / Na 8,19 / P 3,68
gef. C 47,22 / H 4,78 / N 5,12 / Ca 4,70 / Na 8,27 / P 3,80

### Beispiel 10

Calcium(4-),[[(4R)-4-[bis[(carboxy-.kappa.O)methyl]amino-.kappa.N]-6,9-bis((carboxy-.kappa.O)methyl]-1-[(4,4-diphenylcydohexyl)oxy]-1-hydroxy-2-oxa-6,9-diaza-1-phosphaundecan-11-yl-säure-.kappa.N6,.kappa.N9,.kappa.011]1-oxidato(6-)]-,
dimeglumin, dihydrogen

10,0 g (12,88 mmol / 95 % Gehalt ( nach Gewicht ) ) MS-325 Ligand, 0,954g (12,88mmol) Calciumhydroxid und 5,03g ( 25,76 mmol ) Meglumin werden in 200 ml deionisiertem Wasser gelöst und 5 Stunden bei 95°C gerührt. Man läßt abkühlen filtriert über ein 2µ Filter und isoliert das Titelprodukt aus dem Filtrat durch Gefriertrocknung als farbloses amorphes Pulver .
Ausbeute : 17,22 g (quantitativ) Wassergehalt :12,8 %
Elementaranalyse (berechnet auf wasserfreie Substanz )
ber. C 48,41 / H 6,57 / N 6,01 / Ca 3,44 / P 2,66
gef. C 48,28 / H 6,69 / N 6,12 / Ca 3,52 / P 2,77

### Beispiel 11

Calcium(4-),[[(4R)4-[bis((carboxy-.kappa.O)methyl]amino-.kappa.N]-6,9-bis[(carboxy-.kappa.O)methyl]-1-[(4,4-diphenylcyclohexyl )oxy]-1-hydroxy-2-oxa-6,9-diaza-1-phosphaundecan-11-yl-säure-.kappa.N6,.kappa.N9,.kappa.011]1-oxidato(6-)]-,
trimeglumin , monohydrogen

10,0 g (12,88 mmol / 95 % Gehalt ( nach Gewicht ) ) MS-325 Ligand, 1,288g (12,88mmol) Calciumcarbonat und 7,54g ( 38,64 mmol ) Meglumin werden in 200 ml deionisiertem Wasser gelöst und 5 Stunden bei 95°C gerührt. Man läßt abkühlen filtriert über ein 2µ Filter und isoliert das Titelprodukt aus dem Filtrat durch Gefriertrocknung als farbloses amorphes Pulver.
Ausbeute : 19,70 g (quantitativ) Wassergehalt :11,0 %
Elementaranalyse (berechnet auf wasserfreie Substanz)
ber. C 47,64 / H 6,89 / N 6,17/ Ca 2,94 / P 2,28 gef. C 47,80 / H 6,97 / N 6,28 / Ca 3,00 / P 2,40

### Beispiel 12

Calcium(4-),[[(4R)-4-[bis[(carboxy-.kappa.O)methyl]amino-.kappa.N]-6,9-bis[(carboxy-.kappa.O)methyl]-1-[(4,4-diphenytcyclohexyl)oxy]-1-hydroxy-2-oxa-6,9-diaza-1-phosphaundecan-11-yi-säure-.kappa.N6,.kappa.N9,.kappa.011]1-oxidato(6-)]-,
natrium 3,5 , hydrogen 0,5

10,0 g (12,88 mmol / 95 % Gehalt ( nach Gewicht ) MS-325 Ligand 1,288 g (12,88 mmol) Calciumcarbonat und 1,546g 38,64 mmol ) Natriumhydroxid werden in 200 ml deionisiertem Wasser gelöst und 5 Stunden bei 95°C gerührt. Man läßt abkühlen und stellt durch Zugabe einer 5% iger aqu.Lösung von Natriumhydroxid auf pH 7,4 ein. Es wird filtriert und die Titelverbindung durch Gefriertrocknung isoliert.
Ausbeute : 12,01 g (quantitativ ) eines farblosen Pulvers Wassergehalt : 8,6 %
Elementaranalyse : berechnet auf wasserfreie Substanz ) berechnet als 3,5 Natriumsalz
ber C 46,68 / H 4,55 / N 4,93 / Ca 4,70 / Na 9,44 / P 3,63
gef. C 46,61 / H 4,43 / N 5,02 / Ca 4,81 / Na 9,51 / P 3,71

### Beispiel 13

Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
meglumin 3,5 , hydrogen 0,5

10,0 g (12,88 mmol / 95 % Gehalt ( nach Gewicht ) MS-325 Ligand 1,288 g (12,88 mmol) Calciumcarbonat und 7,54g (38,64 mmol ) Meglumin werden in 200 ml deionisiertem Wasser gelöst und 5 Stunden bei 95°C gerührt. Man läßt abkühlen und stellt durch Zugabe einer 5% iger aqu.Lösung von Meglumin auf pH 7,4 ein. Es wird filtriert und die Titelverbindung durch Gefriertrocknung isoliert.
Ausbeute : 20,82 g (quantitativ ) eines farblosen Pulvers Wassergehalt: 9,7 %
Elementaranalyse : berechnet auf wasserfreie Substanz )berechnet als 3,5 Megluminsalz
ber C 47,32 / H 7,04 / N 6,24 / Ca 2,75 / P 2,12
gef. C 47,48 / H 7,15 / N 6,36 / Ca 2,87 / P 2,17

### Beispiel 14

### Herstellung einer Formulierung des Gadolinium-Komplexes von MS-325 als Natrium Salz (200mM ) ( 5 % Komplexbildnerüberschuss der Titelverbindung aus Beispiel 1)

18,12 g (50 mmol ) Gadoliniumoxid , 77,65 (100 mmol / 95 % Gehalt (nach Gewicht) MS-325 , 4.318 g (5,0 mmol ) der Titelverbinaung aus Beispiel 1 una 11,2 g (280mmol ) Natriumhydroxid werden in 350 ml Tris-HCI-Puffer (10mmol / I ) pH 7,4 und 6 Stunden bei 95°C gerührt. Man läßt auf Raumtemperatur abkühlen und stellt mit 20 % iger aqu. Natriumhydroxid auf pH 7,4. Anschließend wird mit Tris-HCI-Puffer (10 mmol / I / pH 7,4 auf 500 ml Gesamtvolumen aufgefüllt, die Lösung über ein 2p - Filter filtriert und das Filtrat in Vials abgefüllt.

### Beispiel 15

### Herstellung einer Formulierung des Gadolinium-Komplexes von MS-325 als Meglumin Salz (200mM ) ( 5 % Komplexbildnerüberschuss der Titelverbindung aus Beispiel 4)

18,12 g (50 mmol ) Gadoliniumoxid , 77,65 (100 mmol / 95 % Gehalt (nach Gewicht) MS-325, 7,783 g (5,0 mmol ) der Titelverbindung aus Beispiel 4 und 54,66 g (280mmol ) Meglumin werden in 350 ml Tris-HCI-Puffer (10mmol /1 )pH 7,4 und 6 Stunden bei 95°C gerührt. Man läßt auf Raumtemperatur abkühler und stellt mit 20 % iger aqu.Meglumin auf pH 7,4. Anschließend wird mit Tris-HCI-Puffer (10 mmol / I/ / pH 7,4 auf 500 ml Gesamtvolumen aufgefüllt, die Lösung über ein 2p - Filter filtriert und das Filtrat in Vials abgefüllt.

### Beispiel 16

### Herstellung einer Formulierung des Gadolinium-Komplexes von MS-325 als Natrium Salz (200mM ) (5 % Komplexbildnerüberschuss der Titelverbindung aus Beispiel 7)

18,12 g (50 mmol ) Gadoliniumoxid, 77,65 (100 mmol / 95 % Gehalt (nach Gewicht) MS-325, 3,879 g (5,0 mmol ) der Titelverbindung aus Beispiel 7 und 11,2 g (280mmol ) Natriumhydroxid werden in 350 ml Tris-HCI-Puffer (10mmol / 1, pH 7,4) und 6 Stunden bei 95°C gerührt. Man läßt auf Raumtemperatur abkühlen und stellt mit 20 % iger aqu. Natriumhydroxid auf pH 7,4. Anschließend wird mit Tris-HCI-Puffer (10 mmol / I/ / pH 7,4 auf 500 ml Gesamtvolumen aufgefüllt, die Lösung über ein 2µ - Filter filtriert und das Filtrat in Vials abgefüllt.

### Beispiel 17

### Herstellung einer Formulierung des Gadolinium-Komplexes von MS-325 als Natrium Salz (200mM ) ( 5 % Komplexbildnerüberschuss der Titelverbindung aus Beispiel 8)

18,12 g (50 mmol ) Gadoliniumoxid , 77,65 (100 mmol / 95 % Gehalt (nach Gewicht) MS-325 , 4,099 g (5,0 mmol ) der Titelverbindung aus Beispiel 8 und 11,2 g (280mmol ) Natriumhydroxid werden in 350 ml Tris-HCI-Puffer (10mmol / I, pH 7,4) und 6 Stunden bei 95°C gerührt. Man läßt auf Raumtemperatur abkühlen und stellt mit 20 % iger aqu. Natriumhydroxid auf pH 7,4. Anschließend wird mit Tris-HCI-Puffer (10 mmol / I/ pH 7,4 auf 500 ml Gesamtvolumen aufgefüllt, die Lösung über ein 2µ - Filter filtriert und das Filtrat in Vials abgefüllt.

### Beispiel 18

### Herstellung einer Formulierung des Gadolinium-Komplexes von MS-325 als Natrium Salz (200mM ) ( 5 % Komplexbildnerüberschuss der Titelverbindung aus Beispiel 9)

18,12 g (50 mmol ) Gadoliniumoxid, 77,65 (100 mmol / 95 % Gehalt (nach Gewicht) MS-325, 4,209 g (5,0 mmol ) der Titelverbindung aus Beispiel 9 und 11,2 g (280mmol ) Natriumhydroxid werden in 350 ml Tris-HCI-Puffer (10mmol/l, pH7,4) und 6 Stunden bei 95°C gerührt. Man läßt auf Raumtemperatur abkühlen und stellt mit 20 % iger aqu. Natriumhydroxid auf pH 7,4. Anschließend wird mit Tris-HCI-Puffer (10 mmol / I/ / pH 7,4 auf 500 ml Gesamtvolumen aufgefüllt, die Lösung über ein 2µ - Filter filtriert und das Filtrat in Vials abgefüllt.

### Beispiel 19

### Herstellung einer Formulierung des Gadolinium-Komplexes von MS-325 als Natrium Salz (200mM ) ( 5 % Komplexbildnerüberschuss der Titelverbindung aus Beispiel 12)

18,12 g (50 mmol ) Gadoliniumoxid, 77,65 (100 mmol / 95 % Gehalt (real Gewicht) MS-325, 4,26 g (5,0 mmol ) der Titelverbindung aus Beispiel 12 und 11,2 g (280mmol ) Natriumhydroxid werden in 350 ml Tris-HCI-Puffer (10mmol/l, pH7,4) und 6 Stunden bei 95°C gerührt. Man läßt auf Raumtemperatur abkühlen und stellt mit 20 % iger aqu. Natriumhydroxid auf pH 7,4. Anschließend wird mit Tris-HCI-Puffer (10 mmol / I/ / pH 7,4 auf 500 ml Gesamtvolumen aufgefüllt, die Lösung über ein 2µ - Filter filtriert und das Filtrat in Vials abgefüllt.

### Beispiel 20

### Herstellung einer Formulierung des Gadolinium-Komplexes von MS-325 als Meglumin Salz (200mM ) ( 5 % Komplexbildnerüberschuss der Titelverbindung aus Beispiel 13)

18,12 g (50 mmol ) Gadoliniumoxid, 77,65 (100 mmol / 95 % Gehalt (nach Gewicht) MS-325, 7,297 g (5,0 mmol ) der Titelverbindung aus Beispiel 13 und 54,66 g (280mmol ) Meglumin werden in 350 ml Tris-HCI-Puffer (10mmol /l) pH 7,4 und 6 Stunden bei 95°C gerührt. Man läßt auf Raumtemperatur abkühlen und stellt mit 20 % iger aqu.Meglumin auf pH 7,4. Anschließend wird mit Tris-HCI-Puffer (10 mmol / I/ pH 7,4 auf 500 ml Gesamtvolumen aufgefüllt, die Lösung über ein 2µ - Filter filtriert und das Filtrat in Vials abgefüllt.

### Beispiel 21

### Herstellung einer Formulierung des Gadolinium-Komplexes von MS-325 als Meglumin Salz (200mM ) ( 5 % Komplexbildnerüberschuss der Titelverbindung aus Beispiel 10)

18,12 g (50 mmol ) Gadoliniumoxid, 77,65 (100 mmol / 95 % Gehalt (nach Gewicht) MS-325, 5,83 g (5,0 mmol ) der Titelverbindung aus Beispiel 10 und 54,66 g (280mmol ) Meglumin werden in 350 ml Tris-HCI-Puffer (10mmol /l ) pH 7,4 und 6 Stunden bei 95°C gerührt. Man läßt auf Raumtemperatur abkühlen und stellt mit 20 % iger aqu.Meglumin auf pH 7,4. Anschließend wird mit Tris-HCl-Puffer (10 mmol / I/ / pH 7,4 auf 500 ml Gesamtvolumen aufgefüllt, die Lösung über ein 2µ - Filter filtriert und das Filtrat in Vials abgefüllt.

### Beispiel 22

### Herstellung einer Formulierung des Gadolinium-Komplexes von MS-325 als Natrium Salz (200mM ) ( 5 % Komplexbildnerüberschuss der Titelverbindung aus Beispiel 1)

18,12 g (50 mmol ) Gadoliniumoxid , 77,65 (100 mmol / 95 % Gehalt (nach Gewicht) MS-325, 1,08 g (1,25 mmol ) der Titelverbindung aus Beispiel 1 und 11,2 g (280mmol ) Natriumhydroxid werden in 350 ml Tris-HCl-Puffer (10mmol/l, pH7,4) und 6 Stunden bei 95°C gerührt. Man läßt auf Raumtemperatur abkühlen und stellt mit 20 % iger aqu. Natriumhydroxid auf pH 7,4. Anschließend wird mit Tris-HCI-Puffer (10 mmol / I/ pH 7,4 auf 500 ml Gesamtvolumen aufgefüllt, die Lösung über ein 2µ - Filter filtriert und das Filtrat in Vials abgefüllt.

### Beispiel 23

### Herstellung einer Formulierung des Gadolinium-Komplexes von MS-325 als Natrium Salz (200mM ) ( 2,5 % Komplexbildnerüberschuss der Titelverbindung aus Beispiel 1)

18,12 g (50 mmol ) Gadoliniumoxid , 77,65 (100 mmol / 95 % Gehalt (nach Gewicht) MS-325, 2,16 g (2,50 mmol ) der Titelverbindung aus Beispiel 1 und 11,2 g (280mmol ) Natriumhydroxid werden in 350 ml Tris-HCI-Puffer (10mmol/l, pH7,4) und 6 Stunden bei 95°C gerührt. Man läßt auf Raumtemperatur abkühlen und stellt mit 20 % iger aqu. Natriumhydroxid auf pH 7,4. Anschließend wird mit Tris-HCI-Puffer (10 mmol / I/ pH 7,4 auf 500 ml Gesamtvolumen aufgefüllt, die Lösung über ein 2µ - Filter filtriert und das Filtrat in Vials abgefüllt.

### Beispiel 24

### Herstellung einer Formulierung des Gadolinium-Komplexes von MS-325 als Meglumin Salz (200mM ) ( 1,25 % Komplexbildnerüberschuss der Titelverbindung aus Beispiel 4)

18,12 g (50 mmol ) Gadoliniumoxid, 77,65 (100 mmol / 95 % Gehalt (nach Gewicht) MS-325, 1,946 g (1,25 mmol ) der Titelverbindung aus Beispiel 4 und 54,66 g (280mmol ) Meglumin werden in 350 ml Tris-HCI-Puffer (10mmol /l) pH 7,4 und 6 Stunden bei 95°C gerührt. Man läßt auf Raumtemperatur abkühlen und stellt mit 20 % iger aqu.Meglumin auf pH 7,4. Anschließend wird mit Tris-HCl-Puffer (10 mmol /l, pH 7,4 auf 500 ml Gesamtvolumen aufgefüllt, die Lösung über ein 2µ - Filter filtriert und das Filtrat in Vials abgefüllt.

### Beispiel 25

### Herstellung einer Formulierung des Gadolinium-Komplexes von MS-325 als Meglumin Salz (200mM ) ( 2,5 % Komplexbildnerüberschuss der Titelverbindung aus Beispiel 4)

18,12 g (50 mmol ) Gadoliniumoxid , 77,65 (100 mmol / 95 % Gehalt (nach Gewicht) MS-325, 3,891 g (2,5 mmol ) der Titelverbindung aus Beispiel 4 und 54,66 g (280mmol ) Meglumin werden in 350 ml Tris-HCI-Puffer (10mmol /1, pH 7,4 und 6 Stunden bei 95°C gerührt. Man läßt auf Raumtemperatur abkühlen und stellt mit 20 % iger aqu.Meglumin auf pH 7,4. Anschließend wird mit Tris-HCI-Puffer (10 mmol / I/ pH 7,4 auf 500 ml Gesamtvolumen aufgefüllt, die Lösung über ein 2µ - Filter filtriert und das Filtrat in Vials abgefüllt.

### Beispiel 26

### Herstellung einer Formulierung des Gadolinium-Komplexes von MS-325 als Natrium Salz (200mM ) ( 5 % Komplexbildnerüberschuss der Titelverbindung aus Beispiel 1)

### Alternativ-Verfahren

18,12 g (50 mmol ) Gadoliniumoxid , 77,65 (100 mmol / 95 % Gehalt (nach Gewicht) MS-325, 11,2g (280 mmol) Natriumhydroxid werden in 350 ml Tris-HCl-Puffer (10mmol/l, pH7,4) und 6 Stunden bei 95°C gerührt. Man läßt auf Raumtemperatur abkühlen, gibt 4,318g (5,0mmol ) der Titelverbindung aus Beispiel 1 zu und stellt mit 20% iger aqu. Natriumhydroxid auf pH 7,4. Anschließend wird mit Tris-HCl-Puffer (10mmol / pH 7,4) 500 ml Gesamtvolumen aufgefüllt, die Lösung über ein 2µ Filter filtriert und das Filtrat in Vials abgefüllt.

### Beispiel 27

### Herstellung einer Formulierung des Gadolinium-Komplexes von MS-325 als Meglumin Salz (200mM ) ( 5 % Komplexbildnerüberschuss der Titelverbindung aus Beispiel 4)

### Alternativ-Verfahren

18,12 g (50 mmol ) Gadoliniumoxid , 77,65 (100 mmol / 95 % Gehalt (nach Gewicht) MS-325, und 54,66g (280 mmol) Meglumin werden in 350 ml Tris-HCl-Puffer (10mmol/l, pH7,4) und 6 Stunden bei 95°C gerührt. Man läßt auf Raumtemperatur abkühlen, gibt 7,783g (5,0mmol ) der Titelverbindung aus Beispiel 4 zu und stellt mit 20%iger aqu.Meglumin auf pH 7,4. Anschließend wird mit Tris-HCI-Puffer (10mmol / I pH 7,4) 500 ml Gesamtvolumen aufgefüllt, die Lösung über ein 2µ Filter filtriert und das Filtrat in Vials abgefüllt.

### Beispiel 28

### Herstellung einer Formulierung des Gadolinium-Komplexes von MS-325 als Natrium Salz (200mM ) (2,50 % Komplexbildnerüberschuss der Titelverbindung aus Beispiel 1)

### Alternativ-Verfahren

18,12 g (50 mmol ) Gadoliniumoxid , 77,65 (100 mmol / 95 % Gehalt (nach Gewicht) MS-325, 11,2g (280mmol) Natriumhydroxid werden in 350 ml Tris-HCI-Puffer (10mmol/l, pH7,4) und 6 Stunden bei 95°C gerührt. Man läßt auf Raumtemperatur abkühlen, gibt 2,16g (2,50mmol) der Titelverbindung aus Beispiel 1 zu und stellt mit 20%iger aqu. Natriumhydroxid auf pH 7,4. Anschließend wird mit Tris-HCI-Puffer (10mmol / I pH 7,4) 500 ml Gesamtvolumen aufgefüllt, die Lösung über ein 2µ Filter filtriert und das Filtrat in Vials abgefüllt.

### Beispiel 29

### Herstellung einer Formulierung des Gadolinium-Komplexes von MS-325 als Meglumin Salz (200mM ) ( 2,50 % Komplexbildnerüberschuss der Titelverbindung aus Beispiel 4)

### Alternativ-Verfahren

18,12 g (50 mmol ) Gadoliniumoxid , 77,65 (100 mmol / 95 % Gehalt (nach Gewicht) MS-325, und 54,66g (280 mmol) Meglumin werden in 350 ml Tris-HCI-Puffer (10mmol/l, pH7,4) und 6 Stunden bei 95°C gerührt. Man läßt auf Raumtemperatur abkühlen, gibt 3,891g (2,50mmol) der Titelverbindung aus Beispiel 4 zu und stellt mit 20%iger aqu. Meglumin auf pH 7,4. Anschließend wird mit Tris-HCI-Puffer (10mmol / I pH 7,4) 500 ml Gesamtvolumen aufgefüllt, die Lösung über ein 2µ Filter filtriert und das Filtrat in Vials abgefüllt.

### Beispiel 30

### ¹H und ³¹P NMR Spektren der Verbindungen aus den Beispielen 1 und 2

Alle Messungen wurden an einem AMX 400 NMR-Spektrometer (400MHz, Bruker) durchgeführt.

¹H chemical shifts sind in δ (ppm) relativ zum Lösungsmittel (D₂O δ = 4,8 ppm) angegeben.

³¹P chemical shifts sind in δ (ppm) relativ zum externen Standard H₃PO₄ (85%, δ = 0 ppm) angegeben.

Titelverbindungen aus Beispiel 1 und 2 wurden in D₂O gelöst und das Spektrum bei Raumtemperatur aufgenommen.

### Ergebnis:

Titelverbindung aus Beispiel 1
- 1H:: 1.4-1.7 (m, 2H), 1.8-2.1 (m, 3H), 2.15-2.25 (m, 2H), 2.32 (t 12Hz, 1H). 2.5-2.92 (m, 7H), 2.95-3.4 (m, 8H), 3.45 (d 16 Hz, 1H), 3.66 (d 16 Hz, 1H) 3.8-3.95 (m, 2H), 4.23 (m, 1H), 7.15-7.25 (m, 2H), 7.25-7.5 (m, 8H)
- 31P:: 0.38 (q, 6Hz), 0.51 (q, 6Hz)

Titelverbindung aus Beispiel 2
¹H: 1.20-1.45 (breit, 2H), 1.5-1.7 (breit, 2H), 1.75-2.05 (m, 3H), 2.1-2.5 (m, 6H), 2.65-3.20 (m, 8H), 3.24 (d 16Hz, b 1H), 3.45 (d 16 Hz, 1H), 3.50-3.80 (m, 4H), 4.02 (m, 1H), 6.75-7.20 (m, 10H)
³¹P: 0.25 (q, 6Hz), 0.10 (q, 5Hz)

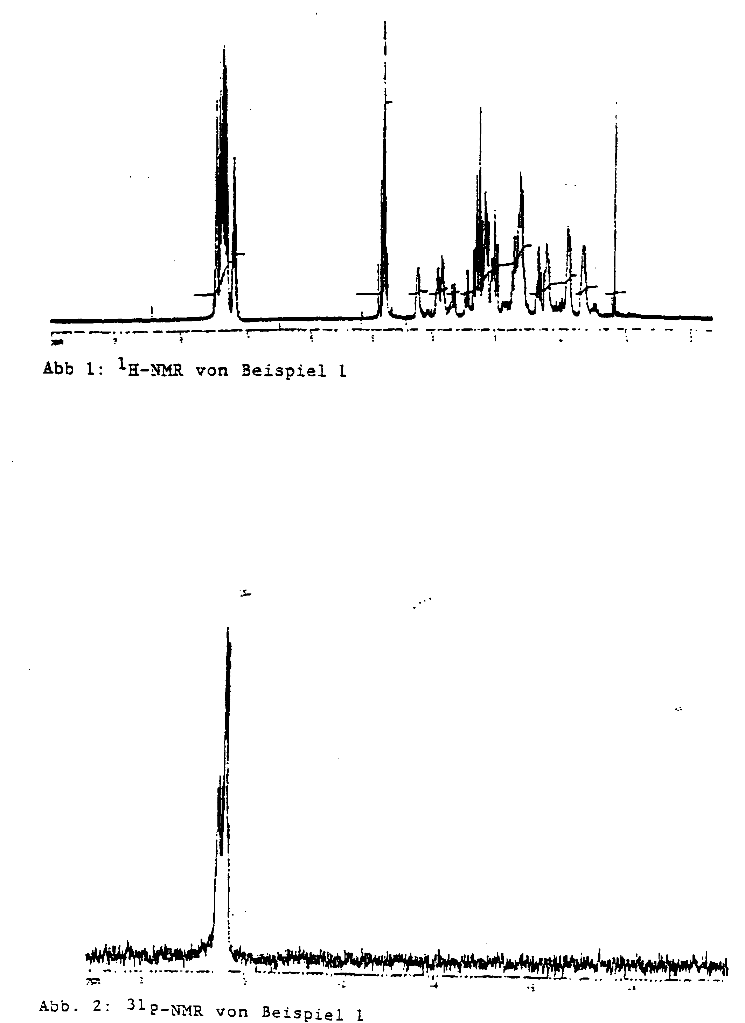

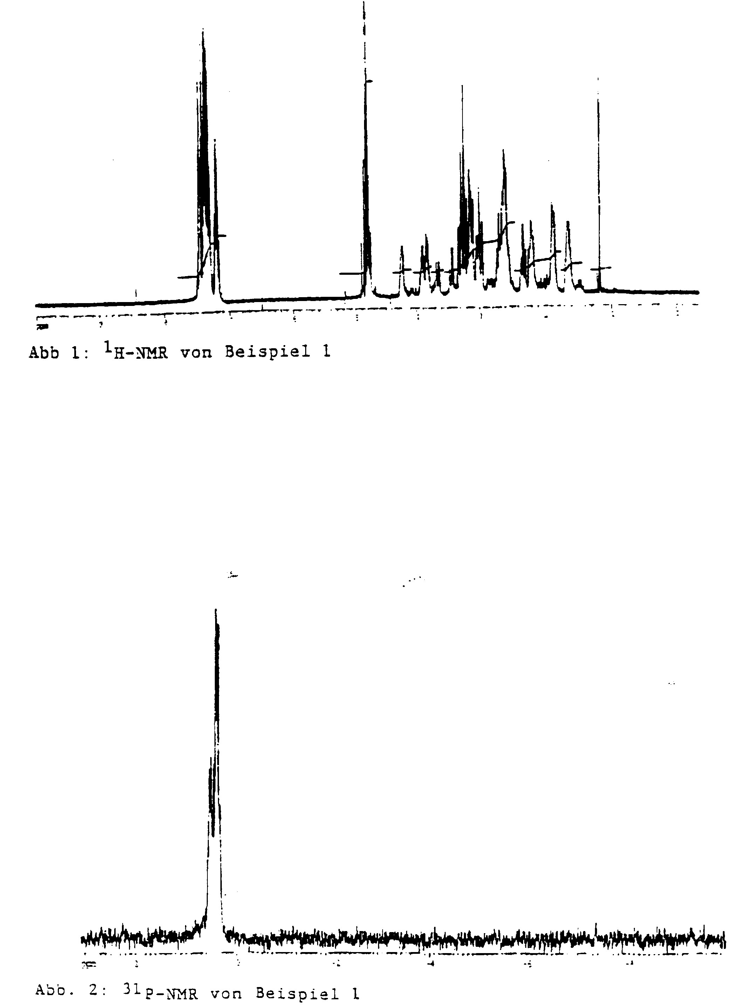

## Patentansprüche

1. Calcium-Komplex von [[ (4R) - 4 - [bis[ (carboxy-.kappa.O) methyl]amino-.kappa.N]-6,9-bis[( carboxy-.kappa.O)methyl]-1-[(4,4- diphenylcyclohexyl )oxyl]-1-hydroxy-2-oxa-6,9-diaza-1-phosphaundecan-11-yl-säure-.kappa.N6, .kappa. N9, .kappa.011]1-oxidato (6-) ]-hexahydrogen (MS-325) sowie dessen Salze mit physiologisch verträglichen Kationen

2. Verbindungen gemäß Anspruch 1, dadurch gekennzeichnet, daß mindestens eines der physiologisch verträglichen Kationen Natrium, Calcium, Kalium, Meglumin, Ethanolamin, Diethanolamin, Morpholin, Glucamin, Dimethylglucamin, Lysin, Arginin oder Ornithin ist.

3. Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
tetranatrium
Calcium(4-),[[(4R)-4-[bis[(carboxy-.kappa.O)methyl]amino-.kappa.N]-6,9-bis[(carboxy-.kappa.0)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
calcium, dinatrium
Calcium(4-),[[(4R)-4-[bis[(carboxy-.kappe.O)methyl]amino-.kappa.N]-6,9-bis[(carboxy-.kappa.O)methyl]-1-((4,4-diphenylcyclöhexyl)oxy]-1-hydroxy-2-oxa-6,9-d1aza-1-phosphaundecan-11-yl-säure-.kappa,N6,.kappa.N9,.kappa.011]1-oxidato(6-)]-,
dicalcium
Calcium(4-),[[(4R)4-[bis[(carboxy-,kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
tetrameglumin
Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa,N6,.kappa.N9,.kappa.011]1-oxidato(6-)]-,
calcium, dimeglumin
Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
calcium, dihydrogen
Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
tetrahydrogen
Calcium(4-),[[(4R)-4-[bis[(carboxy-.kappa.O)methyl]amino-.kappa.N]-6,9-bis[(carboxy-.kappa.0)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
dinatrium, dihydrogen
Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
trinatrium, monohydrogen
Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl )oxy]-1-hydroxy-2-oxa-6,9-diaza-1-phosphaundecan-11-yl-säure-.kappa.N6,.kappa.N9,.kappa.011]1-oxidato(6-)]-,
dimeglumin, dihydrogen
Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
trimeglumin, monohydrogen
Calcium(4-),[[(4R)-4-[bis[(carboxy-.kappa.O)methyl]amino-.kappa,N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
natrium 3,5, hydrogen 0,5
Calcium(4-),[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,
meglumin 3,5 , hydrogen 0,5

4. Pharmazeutische Mittel enthaltend mindestens eine physiologisch verträgliche Verbindung nach einem der Ansprüche 1 - 3, gegebenenfalls mit den in der Galenik üblichen Zusätzen.

5. Verfahren zur Herstellung einer galenischen Formulierung von Gd-MS-325, dadurch gekennzeichnet, daß man Gadoliniumoxid,
[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-, hexahydrogen, physiologisch verträglichen Puffer und anorganische Base mit einem Calciumkomplex nach Anspruch 1 umsetzt.

6. Verfahren zur Herstellung einer galenischen Formulierung von Gd-MS-325, dadurch gekennzeichnet, daß man Gadoliniumoxid,
[[(4R)-4-[bis[(carboxy-.kappa.O)methyl]amino-.kappa.N]-6,9-bis[(carboxy-.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-, hexahydrogen,
physioloigsch verträglichen Puffer und organische Base mit einem Calciumkomplex nach Anspruch 1 umsetzt.

7. Verfahren zur Herstellung einer galenischen Formulierung von Gd-MS-325, dadurch gekennzeichnet, daß man Gadoliniumoxid und ein Salz von
[[(4R)-4-[bis[(carboxy-.kappa.O)methyl]amino-.kappa.N]-6,9bis[(carboxy.kappa.O)methyl]-1-[(4,4-diphenylcyclohexyl)oxy]-1-hydroxy-2-oxa-6,9-diaza-1-phosphaundecan-11-yl-säure-.kappa.N6,.kappa.N9,.kappa.011]1-oxidato(6-)]-,hexahydrogenTris-Puffer und Natriumoxid zu Gadolinium(3-),[[4-[bis[(carboxy-.kappa.0)methyl]amino-.kappa.N]-6,9-bis[(carboxy-.kappe.O)methyl]-1-[(4,4-diphenylcydohexyt)oxyl-1-hydroxy-2-oxa-6,9-diaza-1-phosphaundecan-11-yl-säure-.kappa.N6,.kappa.N9,.kappa.011]oxidato(6-)]
umsetzt und dann mit einem Calciumkomplex gemäß Anspruch 1 versetzt.

8. Pharmazeutisches Mittel gemäß Anspruch 4, dadurch gekennzeichnet, daß die Formulierung Gd-MS-325 sowie einen Anteil eines Calciumkomplexes gemäß Anspruch 1, der zwischen 0,05-15 Mol% bezogen auf Gd-MS-325 liegt, enthält

9. Pharmazeutisches Mittel gemäß Anspruch 4, dadurch gekennzeichnet, daß die Formulierung Gd-MS-325 sowie einen Anteil eines Calciumkomplexes gemäß Anspruch 1, der zwischen 0,5-5 Mol% bezogen auf Gd-MS-325 liegt, enthält

10. Pharmazeutisches Mittel gemäß Anspruch 8, dadurch gekennzeichnet, daß die Lösung 200 mmolar an Gd-MS-325 ist.

11. Verwendung von mindestens einer physiologisch verträglichen Verbindung nach Anspruch 1 für die Herstellung von Mitteln zur Verringerung von Wirkungen, die durch Schwermetalle verursacht werden.

12. Verwendung von mindestens einer physiologisch verträglichen Verbindung nach Anspruch 1 für die Herstellung von Mitteln für die NMR- und/oder Röntgen-Diagnostik.

## Claims

1. Calcium complex of [[(4R)-4-[bis[(carboxy-kappa.O)methyl]amino.kappa.N]-6,9-bis[(carboxy .kappa.O)-methyl]-1-[(4,4-diphenylcyclohexyl)oxy]-1-hydroxy-2-oxa-6,9-diaza-1-phosphaundecan-ll-yl acid .kappa.N6, .kappa.N9,.kappa.011]1-oxidato(6-)] hexahydrogen (MS-325) and its salts with physiologically acceptable cations.

2. Compounds according to Claim 1, characterized in that at least one of the physiologically acceptable cations is sodium, calcium, potassium, meglumine, ethanolamine, diethanolamine, morpholine, glucamine, dimethylglucamine, lysine, arginine or ornithine.

3. Calcium(4-),[[(4R)-4-[bis[(carboxy.kappa.O)-methyl]amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,.kappa.N9,-.kappa.011]1-oxidato(6-)]-,
tetrasodium
calcium(4-),[[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9,.kappa.011]1-oxidato(6-)]-,
calcium, disodium
calcium(4-), [[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9,.kappa.011]1-oxidato(6-)]-,
dicalcium
calcium(4-),[[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9,.kappa.011]1-oxidato(6-)1-,
tetrameglumine
calcium(4-), [[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-ll-yl acid .kappa.N6,-.kappa.N9, .kappa.011]1-oxidato(6-)]-,
calcium, dimeglumine
calcium(4-),[[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9,.kappa.011]1-oxidato(6-)]-,
calcium, dihydrogen
calcium(4-), [[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9, .kappa.O11]1-oxidato(6-)]-,
tetrahydrogen
calcium(4-), [[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9,.kappa.011]1-oxidato(6-)]-,
disodium, dihydrogen
calcium(4-), [[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9,.kappa.011]1-oxidato(6-)]-,
trisodium, monohydrogen
calcium(4-),[[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-ll-yl acid .kappa.N6,-.kappa.N9, .kappa.011]1-oxidato(6-)]-,
dimeglumine, dihydrogen
calcium(4-),[[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9,.kappa.011]1-oxidato(6-)]-,
calcium(4-),[[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9,.kappa.011]1-oxidato(6-)]-,
sodium 3.5, hydrogen 0.5
calcium (4-),[[(4R)-4-[bis[(carboxy.kappa.O)methyl]-amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,-.kappa.N9, .kappa.011]1-oxidato(6-)]-,
meglumine 3.5, hydrogen 0.5

4. Pharmaceutical compositions, comprising at least one physiologically acceptable compound according to any of Claims 1-3, if appropriate with the additives customary in pharmaceutical technology.

5. Process for preparing a pharmaceutical formulation of Gd-MS-325, characterized in that gadolinium oxide,
[[(4R)-4-[bis[(carboxy.kappa.O)methyl]amino.kappa.N]-6,9-bis[(carboxy.kappa.O)methyl]-1-[(4,4-diphenylcyclo-hexyl)oxy]-1-hydroxy-2-oxa-6,9-diaza-1-phosphaundecan-11-yl acid .kappa.N6,.kappa.N9,.kappa.011]1-oxidato-(6-)]-,
hexahydrogen, physiologically acceptable buffer and inorganic base are reacted with a calciuum complex according to Claim 1.

6. Process for preparing a pharmaceutical formulation of Gd-MS-325, characterized in that gadolinium oxide,
[[(4R)-4-[bis[(carboxy.kappa.O)methyl]amino.kappa.N]-6,9-bis((carboxy.kappa.O)methyl]-1-[(4,4-diphenylcyclohexyl)oxy]-1-hydroxy-2-oxa-6,9-diaza-1-phosphaundecan-11-yl acid .kappa.N6,.kappa.N9,.kappa.011]1-oxidato-(6-)]-,
hexahydrogen, physiologically acceptable buffer and inorganic base are reacted with a calciuum complex according to Claim 1.

7. Process for preparing a pharmaceutical formulation of Gd-MS-325, characterized in that gadolinium oxide and a salt of
[[(4R)-4-[bis[(carboxy.kappa.O)methyl]amino.kappa.N]-6,9-bis[(carboxy.kappa.O)methyl]-1-[(4,4-diphenylcyclohexyl)oxy]-1-hydroxy-2-oxa-6,9-diaza-1-phosphaundecan-11-yl acid .kappa.N6,.kappa.N9,.kappa.011]1-oxidato-(6-)]-, hexahydrogen Tris-buffer and sodium oxide are reacted to give gadolinium(3-),[[4-[bis[(carboxy.kappa.-O)methyl]amino.kappa.N]-6,9-bis[(carboxy.kappa.O)-methyl]-1-[(4,4-diphenylcyclohexyl)oxy]-1-hydroxy-2-oxa-6,9-diaza-1-phosphaundecan-11-yl acid .kappa.N6, .kappa.N9,.kappa.011]oxidato(6-)] which is then admixed with a calcium complex according to Claim 1.

8. Pharmaceutical composition as claimed in Claim 4, characterized in that the formulation comprises Gd-MS-325 and an amount of a calcium complex according to Claim 1 which is 0.05-15 mol%, based on Gd-MS-325.

9. Pharmaceutical composition according to Claim 4, characterized in that the formulation comprises Gd-MS-325 and an amount of a calcium complex according to Claim 1 which is 0.5-5 mol%, based on Gd-MS-325.

10. Pharmaceutical composition according to Claim 8, characterized in that the solution is 200 mmolar with respect to Gd-Ms-325.

11. Use of at least one physiologically acceptable compound according to Claim 1 for preparing compositions for reducing effects caused by heavy metals.

12. Use of at least one physiologically acceptable compound according to Claim 1 for preparing compositions for NMR and/or X-ray diagnosis.

## Revendications

1. Complexe au calcium de l'acide [[(4R)-4-[bis[(carboxy.kappa.O)méthyl] amino.kappa.N] -6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)]hexahydrogène (MS-325) et ses sels avec des cations physiologiquement acceptables.

2. Composés selon la revendication 1, caractérisés en ce qu'au moins l'un des cations physiologiquement acceptables est le sodium, le calcium, le potassium, la méglumine, l'éthanolamine, la diéthanolamine, la morpholine, la glucamine, la diméthylglucamine, la lysine, l'arginine ou l'ornithine.

3. Calcium (4-), acide [[(4R)-4-[bis[(carboxy.kappa.O)méthyl]amino.kappa.N]-6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)] -,
tétrasodium
calcium(4-),acide[[(4R)-4-[bis[(carboxy.kappa.O)-méthyl] amino.kappa.N] -6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)]-,
calcium, disodium
calcium(4-),acide [[(4R)-4-[bis[(carboxy.kappa.O)-méthyl]amino.kappa.N]-6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)]-,
dicalcium
calcium (4-),acide[[(4R)-4-[bis[(carboxy.kappa.O)-méthyl]amino.kappa.N]-6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9, .kappa.011]1-oxydato(6-)] -,
tétraméglumine
calcium (4-),acide[[(4R) -4- [bis[(carboxy.kappa.O)-méthyl] amino.kappa.N]-6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)]-,
calcium, diméglumine
calcium (4-),acide[[(4R)-4- [bis[(carboxy.kappa.O)-méthyl]amino.kappa.N]-6,9-bis[(carboxy.kappa.O)méthyl]-1-[(4,4-diphénylcyclohexyl)oxy] -1-hydroxy-2-oxa-6,9-diaza-1-phospha-undécan-11-ylique-kappa.N6,-.kappa.N9,.kappa.011]1-oxydato(6-)]-,
calcium, dihydrogène
calcium (4-), acide[[(4R) -4- [bis[(carboxy.kappa.O)-méthyl] amino.kappa.N] -6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,- .kappa.N9, .kappa.011]1-oxydato(6-)]-,
tétrahydrogène
calcium (4-),acide[[(4R) -4- [bis[(carboxy.kappa.O)-méthyl] amino.kappa.N] -6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9, .kappa. 011]1-oxydato(6-)]-,
disodium, dihydrogène
calcium(4-) ,acide[[(4R)-4-[bis[(carboxy.kappa.O)-méthyl]amino.kappa.N]-6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)1-,
trisodium, monohydrogène
calcium(4-),acide[[(4R)-4-[bis[(carboxy.kappa.O)-méthyl] amino.kappa.N] -6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9, .kappa.011]1-oxydato(6-)1-,
diméglumine, dihydrogène
calcium (4-),acide[[(4R) -4- [bis[(carboxy.kappa.O)-méthyl]amino.kappa.N]-6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)]-,
triméglumine, monohydrogène
calcium (4-),acide [[(4R)-4-[bis[(carboxy.kappa.O)-méthyl] amino.kappa.N] -6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)]-,
sodium 3,5, hydrogène 0,5
calcium (4-), acide[[(4R) -4- [bis[(carboxy.kappa.O)-méthyl]amino.kappa.N] -6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,-.kappa.N9,.kappa.011]1-oxydato(6-)] -,
méglumine 3,5, hydrogène 0,5.

4. Compositions pharmaceutiques comprenant au moins un composé physiologiquement acceptable selon l'une des revendications 1-3, éventuellement avec les additifs habituels dans le domaine galénique.

5. Procédé de préparation d'une formulation galénique de Gd-MS-325, caractérisé en ce que l'on fait réagir de l'oxyde de gadolinium,
de l'acide [[(4R) -4- [bis[(carboxy.kappa.O)méthyl]-amino.kappa.N]-6,9-bis[(carboxy.kappa.O)méthyl]-1-[(4,4-diphénylcyclohexyl)oxy] -1-hydroxy-2-oxa-6,9-diaza-1-phospha-undécan-ll-ylique-kappa.N6,.kappa.N9,.kappa.011]1-oxydato-(6-)] hexahydrogène, un tampon physiologiquement acceptable et une base inorganique, avec un complexe au calcium selon la revendication 1.

6. Procédé de préparation d'une formulation galénique de Gd-MS-325, caractérisé en ce que l'on fait réagir de l'oxyde de gadolinium,
de l'acide [[(4R)-4-[bis[(carboxy.kappa.O)méthyl]-amino.kappa.N]-6,9-bis[(carboxy.kappa.O)méthyl]-1-[(4,4-diphénylcyclohexyl)oxy]-1-hydroxy-2-oxa-6,9-diaza-1-phospha-undécan-11-ylique-kappa.N6,.kappa.N9,.kappa.011]1-oxydato-(6-)]hexahydrogène,
un tampon physiologiquement acceptable et une base inorganique, avec un complexe au calcium selon la revendication 1.

7. Procédé de préparation d'une formulation galénique de Gd-MS-325, caractérisé en ce que l'on fait réagir de l'oxyde de gadolinium et un sel de l'acide [[(4R) -4- [bis[(carboxy.kappa.O)méthyl]-amino.kappa.N]6,9-bis[(carboxy.kappa.O)méthyl]-1-[(4,4-diphénylcyclo-hexyl)oxy]-1-hydroxy-2-oxa-6,9-diaza-1-phospha-undécan-11-ylique-kappa.N6,.kappa.N9,.kappa.011]1-oxydato-(6-)]hexahydrogène, du tampon Tris et de l'oxyde de sodium, pour donner du gadolinium(3-),acide[[4-[bis[(carboxy.kappa.O)méthyl]amino.kappa.N] -6,9-bis-[(carboxy.kappa.O)méthyl]-1-[(4,4-diphénylcyclohexyl)-oxy] -1-hydroxy-2-oxa-6,9-diaza-1-phospha-undécan-11-ylique-kappa.N6,.kappa.N9,.kappa.011]oxydato(6-)] qui est alors mélangé avec un complexe au calcium selon la revendication 1.

8. Composition pharmaceutique selon la revendication 4, caractérisée en ce que la formulation comprend du Gd-MS-325 et une proportion d'un complexe au calcium selon la revendication 1, qui est comprise entre 0,05 et 15% en moles, par rapport au Gd-MS-325.

9. Composition pharmaceutique selon la revendication 4, caractérisée en ce que la formulation comprend du Gd-MS-325 et une proportion d'un complexe au calcium selon la revendication 1, qui est comprise entre 0,5 et 5% en moles, par rapport au Gd-MS-325.

10. Composition pharmaceutique selon la revendication 8, caractérisée en ce que la concentration de la solution est de 200 mM par rapport au Gd-MS-325.

11. Utilisation d'au moins un composé physiologiquement acceptable selon la revendication 1 pour la préparation de compositions destinées à réduire les effets causés par les métaux lourds.

12. Utilisation d'au moins un composé physiologiquement acceptable selon la revendication 1 pour la préparation de compositions destinées au diagnostic par RMN et/ou par rayons X.
